# EUROPÄISCHE PATENTSCHRIFT

(11) **EP 1 945 603 B1**
(45) Veröffentlichungstag und Bekanntmachung des Hinweises auf die Patenterteilung: **30.05.2012**
(21) Anmeldenummer: 06819191.5
(22) Anmeldetag: 31.10.2006
(51) Int. Cl.: C07C 209/48, C07C 211/11, B01J 10/00, B01J 8/20, B01D 3/14

(54) **VERFAHREN ZUR SYNTHESE VON N, N-DIMETHYL-1,3-DIAMINOPRAN (DMAPA)**
N, N-DIMETHYL-1,3-DIAMINOPROPANE (DMAPA) SYNTHESISING METHOD
PROCEDE DE SYNTHESE DE N,N-DIMETHYL-1,3-DIAMINOPROPANE (DMAPA)

(30) Priorität: 03.11.2005 DE 102005052457
(43) Veröffentlichungstag der Anmeldung: 23.07.2008
(73) Patentinhaber: BASF SE, 67056 Ludwigshafen (DE)
(72) Erfinder: ERNST, Martin, 69115 Heidelberg (DE); OEHLENSCHLÄGER, Steffen, 67063 Ludwigshafen (DE); KUHNKE, Frank, 67063 Ludwigshafen (DE); ROSS, Karl-Heinz, 67269 Grünstadt (DE); DEEG, Roland, 67269 Grünstadt (DE); MELDER, Johann-Peter, 67459 Böhl-Iggelheim (DE)
(86) Internationale Anmeldenummer: PCT/EP2006/067952
(87) Internationale Veröffentlichungsnummer: WO 2007/051786

(56) Entgegenhaltungen:
- WO-A-2004/060853
- US-A- 4 172 091

## Beschreibung

Die vorliegende Erfindung betrifft Vorrichtungen und Verfahren zur großtechnischen Herstellung eines Diamins ausgehend von einem entsprechenden Alkenylnitril, das mindestens eine C-C-Doppelbindung enthält, sowie die Verwendung der Vorrichtungen.

Diamine stellen eine wichtige chemische Gruppe dar, die in vielfältigster Weise als Ausgangsstoffe, Zwischen- oder Endprodukte zum Einsatz kommen. Beispielsweise sind Diamine wichtige Bausteine bei der Polyamidsynthese und anderen Polykondensationsreaktionen.

Insbesondere 3-Dimethylaminopropylamin (DMAPA, N,N-Dimethyl-1,3-diaminopropan) stellt ein wichtiges Zwischenprodukt für die technische Produktion beispielsweise von Schmierseifen dar. DMAPA dient zudem als Ausgangsprodukt für die Herstellung von Koagulationsmitteln und soll selbst antikorrosive Eigenschaften aufweisen.

Häufig werden Diamine ebenso wie deren Amin-Analoga durch Reduktion von Nitrilen hergestellt. Diese Reaktion ist insbesondere dann von Vorteil, wenn primäre Amine erhalten werden sollen.

So beschreiben beispielsweise WO-A 2004/060853 sowie WO-A 2004/060039 und US4172091 die katalytische Hydrierung von 3-Dimethylaminopropionitril (DMAPN) zu DMAPA.

EP-A 0913388 beschreibt die Hydrierung von Nitrilen zur Herstellung von Aminen mit Hilfe eines Cobaltkatalysators.

Trotz der beschriebenen Methoden zur Reduktion von Nitrilen zu Aminen oder Diaminen bleibt die Frage offen, wie ein entsprechendes Nitril zur Verfügung gestellt werden kann.

Es besteht daher ein Bedarf, ein integrales Verfahren bereitzustellen, das in besonders effizienter Weise, ausgehend von vergleichsweise einfachen Edukten, Diamine zu erzeugen in der Lage ist.

Hierbei sind insbesondere Verfahren von Bedeutung, die in großtechnischem Maßstab eingesetzt werden, da häufig ein einfaches Scale-up von Laborversuchen über den Technikumsmaßstab hinaus nicht zu gewünschten Resultaten führt.

Eine Aufgabe der vorliegenden Erfindung liegt somit darin, ein großtechnisches Verfahren bereitzustellen, das in besonders effizienter Weise die Herstellung von Diaminen erlaubt.

Die Aufgabe wird gelöst durch ein Verfahren zur großtechnischen Herstellung eines Diamins ausgehend von einem entsprechenden Alkenylnitril, das mindestens eine C-C-Doppelbindung enthält, die Schritte enthaltend
(a) Umsetzung des Alkenylnitrils in einem ersten Reaktor mit einem entsprechenden Monoamin unter exothermer Addition des Monoamins an die mindestens eine Doppelbindung zu einem Aminoalkylnitril, wobei das Monoamin sowie Wasser vorgelegt werden und das Alkenylnitril hinzugefahren wird;
(b) Abdampfen nicht umgesetzten Alkenylnitrils und Monoamins zur Anreicherung des Aminoalkylnitril-Produktes im Sumpf des ersten Reaktors;
(c) Überführung des Aminoalkylnitril-Sumpfproduktes aus Schritt (b) in einen zweiten Reaktor;
(d) Batchweise katalytische Hydrierung des in Schritt (c) überführten Aminoalkylnitrils zum Diamin in dem zweiten Reaktor, wobei für den Batch ein für die Hydrierung von Nitrilen zu Aminen geeigneter Katalysator sowie Wasser, das gewünschte Diamin und eine Base vorgelegt, der zweite Reaktor mit Wasserstoff beaufschlagt und das in Schritt (c) überführte Aminoalkylnitril zudosiert wird; und
(e) Gewinnung des Diamins und gegebenenfalls Wiederholung der Schritte (a) bis (e).

Es hat sich nämlich gezeigt, dass ein großtechnisches Verfahren basierend auf den oben genannten Verfahrensschritten in besonders effizienter Weise im Hinblick auf Ausbeute, Selektivität, Energiebilanz, Ökobilanz sowie unter ökonomischen Aspekten und weiteren wichtigen Parametern eingesetzt werden kann.

Dem Verfahren liegt eine zweistufige Synthese eines Diamins zugrunde. Im ersten Schritt wird ein Alkenylnitril, das mindestens eine C-C-Doppelbindung enthält, mit einem primären oder sekundären Monoamin derart umgesetzt, dass das Amin sich an die C-C-Doppelbindung addiert. Das aus dieser Reaktion erhaltene Aminoalkylnitril wird in einem weiteren Schritt mit Hilfe von Wasserstoff zum gewünschten Diamin reduziert.

Im Rahmen der vorliegenden Erfindung bedeuten die Begriffe "entsprechendes Alkenylnitril" sowie "entsprechendes Monoamin", dass diese derart ausgewählt werden, dass nach den oben beschriebenen zwei Syntheseschritten das Diamin mit der gewünschten Strukturformel erhalten wird.

Handelt es sich beispielsweise bei dem Alkenylnitril um Acrylsäurenitril (ACN) und bei dem Monoamin um Dimethylamin (DMA), so erfolgt zunächst eine Umsetzung der Edukte zu 3-Dimethylaminopropionitril (DMAPN). Die anschließende Hydrierung ergibt 3-Dimethylaminopropylamin (DMAPA). Anders ausgedrückt ist für den Fall, dass DMAPA gewünscht ist, klar ersichtlich, dass das entsprechende Alkenylnitril ACN und das entsprechende Monoamin DMA sein müssen.

Das Alkenylnitril ist vorzugsweise ein C₂-C₄-Alken, das gradkettig oder verzweigt sein kann, bei dem ein Wasserstoffatom durch die Cyanogruppe ausgetauscht ist.

Der Begriff C₂-C₄-Alken bedeutet, ein Alken mit zwei bis vier C-Atomen, das mindestens eine C-C-Doppelbindung enthält. Vorzugsweise ist genau eine C-C-Doppelbindung in α,β-Position zur Cyanogruppe vorhanden. Beispiele für C₂-C₄-Alkene sind Ethen, Propen, 1-Buten, 2-Buten, 2-Methylpropen.

Beispiele für Nitrile sind Acrylnitril, But-2-ensäurenitril, Methacrylsäurenitril, Pent-2-ensäurenitril, 2-Ethylacrylsäurenitril, 2-Methylbut-2-ensäurenitril sowie 3-Methylbut-2-ensäurenitril.

Bevorzugt ist ACN.

Bei dem Monoamin handelt es sich bevorzugt um ein primäres oder sekundäres Amin der allgemeinen Formel R¹R²NH, bei dem R¹, R² unabhängig voneinander H oder C₁-C₄-Alkyl mit der Maßgabe sind, dass wenigstens ein Rest R¹, R² nicht Wasserstoff ist.

C₁-C₄-Alkyl bedeutet Methyl, Ethyl, n-Propyl, i-Propyl, 1-n-Butyl, 2-n-Butyl, i-Butyl, t-Butyl.

Bevorzugt ist DMA.

Sofern es sich bei dem Alkenylnitril um ACN und bei dem Monoamin um Dimethylamin handelt, ergibt sich als gewünschtes Diamin DMAPA, was bevorzugt ist.

Im Rahmen der vorliegenden Erfindung bedeutet der Begriff "großtechnische Herstellung", dass bei der Umsetzung des Alkenylnitrils sowie bei der Hydrierung des Aminoalkylnitrils pro Reaktion eine Mindestmenge in der Größenordnung von 100 kg, bevorzugt 1 t, eingesetzt wird.

Schritt (a) des erfindungsgemäßen Verfahrens betrifft die Umsetzung des Alkenylnitrils in einem ersten Reaktor mit einem entsprechenden Monoamin unter exothermer Addition des Monoamins an die mindestens eine Doppelbindung zu einem Aminoalkylnitril, wobei das Monoamin sowie Wasser vorgelegt werden und das Alkenylnitril hinzugefahren wird.

Die Reaktion verläuft vorzugsweise in einer Blasensäule.

Es ist weiterhin bevorzugt, dass auch nach vollständiger Zugabe des Alkenylnitrils das Monoamin im stöchiometrischen Überschuss vorliegt. Da das Monoamin vorgelegt und das Alkenylnitril zum Reaktionsgemisch zugegeben wird, gilt dies ebenfalls für die Dauer der Zugabe des Alkenylnitrils. Hierdurch kann gewährleistet werden, dass die Polymerisation des Alkenylnitrils möglichst unterdrückt wird. Der Überschuss an Monoamin zu Alkenylnitril beträgt vorzugsweise mindestens 1 mol-%, mehr bevorzugt mindestens 2,5 mol-%, weiterhin mehr bevorzugt mindestens 5 mol-% und besonders bevorzugt mindestens 10 mol-%.

Die Umsetzung in Schritt (a) kann unter Siedehitze und unter Rückführung des Monoamins erfolgen. Durch die aufsteigenden Gasblasen im ersten Reaktor kann eine bessere Durchmischung des Reaktionsgemisches erfolgen und die Wärmeabfuhr kann auf besonders ökonomische Art und Weise erfolgen.

Für die Umsetzung in Schritt (a) des erfindungsgemäßen Verfahrens wird neben dem Monoamin, welches das eine Edukt darstellt, Wasser vorgelegt. Hierbei werden vorzugsweise, bezogen auf das Monoamin, höchstens 20 mol-%, mehr bevorzugt höchstens 15 mol-% und besonders bevorzugt nicht mehr als etwa 10 mol-% Wasser eingesetzt.

Aufgrund des vorhandenen Wassers kann dieses als Zwischensieder dienen, was die Entfernung des Monoamins sowie dessen Kondensation beispielsweise an einem Rückflusskühler erleichtert. Weiterhin kann Wasser als Katalysator für die Addition des Monoamins an die Doppelbindung des Alkenylnitrils dienen.

Die exotherme Reaktion bei der Umsetzung des Alkenylnitrils in Schritt (a) kann derart gesteuert werden, dass die Reaktion unter Rückfluss des Monoamins durchgeführt wird. Dies kann beispielsweise durch einen Rückflusskühler bewerkstelligt werden. Neben dem Monoamin geht hierbei zusätzlich Wasser in die Dampfphase, während im Sumpf des ersten Reaktors das gebildete Aminoalkylnitril verbleibt. Die Reaktion wird vorteilhaft derart gefahren, dass die Sumpftemperatur in Schritt (a) unter 130°C, vorzugsweise unter 120°C, mehr bevorzugt unter 100°C, gehalten wird. Dies gilt insbesondere bei der Herstellung von DMAPA.

Weiterhin ist es vorteilhaft, wenn die Sumpftemperatur konstant bleibt. Vorteilhafterweise wird die Sumpftemperatur durch eine temperaturgesteuerte Druckabsenkung konstant und/oder unter der Maximaltemperatur gehalten. Insbesondere kann dies für den Fall der Herstellung von DMAPN durch Druckabsenkung von 5 auf 1 bar (Überdruck) erreicht werden.

Nach vollständiger Zugabe des Alkylennitrils wird typischerweise die Reaktion weitergefahren, um den Umsatz zu vervollständigen.

Das erfindungsgemäße Verfahren ist besonders als integrales Verfahren, das sowohl die Umsetzung eines Alkenylnitrils mit einem Monoamin als auch die anschließende Hydrierung des gebildeten Aminoalkylnitril-Zwischenproduktes beinhaltet, geeignet. Jedoch stellt der Verfahrensschritt der Umsetzung für sich allein genommen bereits ein vorteilhaftes Verfahren dar. Demzufolge ist ein weiterer Aspekt der vorliegenden Erfindung der oben beschriebene Schritt (a) für sich genommen gegebenenfalls mit dem nachfolgend näher erläuterten Schritt (b).

Der Verfahrensschritt (b) des erfindungsgemäßen Verfahrens betrifft das Abdampfen nicht umgesetzten Alkenylnitrils und Monoamins zur Anreicherung des Aminoalkylnitril-Produktes im Sumpf des ersten Reaktors. Nach erfolgter Umsetzung müssen die nicht umgesetzten Edukte entfernt werden. Dies kann durch Erhitzen des Reaktorinhalts erfolgen, wobei überwiegend das Monoamin und teilweise Wasser verdampfen. Hierbei kann sich jedoch ebenfalls ein geringer Anteil des Aminoalkylnitrils in der Dampfphase befinden. Durch das Abdampfen der Edukte erfolgt die Anreicherung des Aminoalkylnitril-Produktes im Sumpf. Die über den Kopf der Apparatur abgetrennten Bestandteile Monoamin, Wasser sowie etwas Aminoalkylnitril können kondensiert werden und zumindest teilweise in einer nachfolgenden Umsetzung wieder für einen Schritt (a) vorgelegt werden. Um dies zu bewerkstelligen, wird typischerweise das Kondensat in einem Behälter zwischengespeichert. Das so im Sumpf erhaltene Aminoalkylnitril weist vorteilhafterweise einen Höchstgewichtsanteil an Monoamin und Wasser von jeweils 5 Gew.-%, vorzugsweise höchstens jeweils 3 Gew.-%, insbesondere höchstens jeweils 2 Gew.-% auf.

Das Aminoalkylnitril wird dann in Schritt (c) des erfindungsgemäßen Verfahrens in einen zweiten Reaktor überführt. Dies kann durch direkte Überführung der mit einer entsprechenden Rohrleitung verbundenen Reaktoren erfolgen. Typischerweise wird jedoch zunächst das Aminoalkylnitril in einem Lagertank zwischengelagert. Ein Vorteil der Erfindung liegt darin, dass das in Schritt (a) des erfindungsgemäßen Verfahrens hergestellte Aminoalkylnitril Zwischenprodukt eine ausreichende Reinheit aufweist, um im nachfolgenden Hydrierschritt eingesetzt zu werden. Es sollte lediglich ein Filtrationsschritt erfolgen, um feste Bestandteile entsprechend abzutrennen. Daher ist eine bevorzugte Ausführungsform des erfindungsgemäßen Verfahrens darin zu sehen, dass bei der Überführung des Aminoalkylnitrils, welches in Schritt (c) entsprechend überführt wird, außer einem Filtrationsschritt keine weiteren Reinigungsschritte erfolgen.

In Schritt (d) des erfindungsgemäßen Verfahrens erfolgt die batchweise katalytische Hydrierung des in Schritt (c) überführten Aminoalkylnitrils zum Diamin in dem zweiten Reaktor, wobei für jeden Batch ein für die Hydrierung von Nitrilen zu Aminen geeigneter Katalysator sowie Wasser, das gewünschte Diamin und eine Base vorgelegt, der zweite Reaktor mit Wasserstoff beaufschlagt und das in Schritt (c) überführte Aminoalkylnitril zudosiert wird.

Das erfindungsgemäße Verfahren umfasst somit zwei Reaktionsschritte. Ebenso ist jedoch bereits das Hydrierverfahren, welches in Schritt (d) beschrieben wird, gegebenenfalls gemeinsam mit einem Schritt zur Gewinnung des Diamins vorteilhaft. Daher ist ein weiterer Aspekt der vorliegenden Erfindung die nachfolgend näher beschriebene batchweise katalytische Hydrierung gegebenenfalls gemeinsam mit dem Schritt der Gewinnung des Diamins.

Das bei der Hydrierung erzielte Diamin muss bei der Hydrierung zumindest in geringen Mengen vorgelegt werden, um einen geregelten Ablauf der Hydrierung zu gewährleisten.

Es ist bevorzugt, dass in Schritt (d) der Katalysator im Reaktionsgemisch zu mindestens 1 Gew.-% bezogen auf das insgesamt zugegebene Aminoalkylnitril vorhanden ist. Vorzugsweise beträgt der Mindestgehalt 1,25 Gew.-%, mehr bevorzugt 1,5 Gew.-%. Eine vergleichsweise hohe Katalysatorkonzentration im Reaktor kann die Selektivität sowie die Lebenszeit des Katalysators erhöhen. Bezogen auf das zu Beginn vorgelegte Gemisch kann der Anteil an Katalysator bis zu 20 Gew.-%, bevorzugt 15 Gew.-% betragen.

Der für die Hydrierung verwendete Katalysator kann ein im Prinzip beliebiger für die Hydrierung von Nitrilen zu Aminen geeigneter Katalysator sein. Ein solcher Katalysator kann Nickel beispielsweise als Raney-Nickel enthalten. Es sind zahlreiche Katalysatoren im Stand der Technik bekannt. Geeignete Katalysatoren sind beispielsweise in EP-A 913 388, WO-A 2004/060039, WO-A 2004/060853, US-A 4 739 120, JP-A 38 21 353, US-A 2 449 035, US-A 4 375 003, EP-A 1 050 527 sowie DE 70877 beschrieben.

Es ist vorteilhaft, wenn die batchweise katalytische Hydrierung in Schritt (d) des erfindungsgemäßen Verfahrens dahingehend durchgeführt wird, dass in dem besagten Schritt für eine oder mehrere der nachfolgenden Chargen (Batches) Katalysatormenge zur im zweiten Reaktor bereits befindlichen Katalysatormenge zugegeben wird. Dies bedeutet, dass nicht nach jedem Batch die gesamte Katalysatormenge ausgetauscht wird. Hierbei kann die Zugabe in bestimmten Intervallen, beispielsweise nach zehn Chargen durchgeführt werden. Es ist jedoch ebenso möglich, dass nach jedem Batch eine Zugabe an Katalysatormenge erfolgt. Vorteilhafterweise wird die gesamte Katalysatormenge nach vergleichsweise häufigen Zyklen ausgetauscht. Vorzugsweise findet der vollständige Austausch des Katalysators nach frühestens 50 Zyklen (Durchführung der Schritt (a) bis (e)) statt. Mehr bevorzugt findet ein vollständiger Katalysatortausch nach frühestens 100, weiterhin mehr bevorzugt nach mindestens 150 Chargen statt.

Zur Durchführung der batchweisen katalytischen Hydrierung in Schritt (d) des erfindungsgemäßen Verfahrens wird der zweite Reaktor mit Wasserstoff beaufschlagt. Weiterhin wird das Aminoalkylnitril als Edukt zugegeben. Vorzugsweise wird die Reaktion in Schritt (d) derart geführt, dass zu jedem Zeitpunkt Wasserstoff im stöchiometrischen Überschuss vorhanden ist. Vorzugsweise beträgt dieser stöchiometrische Überschuss mindestens 5 mol-%, mehr bevorzugt mindestens 15 mol-%, weiterhin mehr bevorzugt mindestens 20 mol-%. Der Wasserstoffüberschuss kann durch kontinuierlichen oder diskontinuierlichen Druck nachgepresst werden.

Bei der Reaktion in Schritt (d) des erfindungsgemäßen Verfahrens wird eine Base eingesetzt. Vorzugsweise ist diese Base ein Alkalimetallhydroxid, insbesondere Kaliumhydroxid, Natriumhydroxid sowie Mischungen davon.

Nach Beendigung der Hydrierung erfolgt als Schritt (e) des erfindungsgemäßen Verfahrens die Gewinnung des gewünschten Diamins sowie gegebenenfalls die Wiederholung der Schritte (a) bis (e).

Die Gewinnung des Diamins in Schritt (e) kann destillativ erfolgen.

Es ist sinnvoll, dass vor der Destillation das Reaktionsprodukt filtriert und in eine Destillationskolonne überführt wird. Hierbei hat es sich als vorteilhaft erwiesen, dass vor der Filtration der Hauptteil des Katalysators sedimentativ abgetrennt wird. Weiterhin hat sich bei der Destillation als vorteilhaft erwiesen, wenn eine Vorlauffraktion hauptsächlich bestehend aus Wasser und Monoamin bei der Destillation in den ersten Reaktor zurückgeführt wird. Sofern eine Vorlauffraktion hauptsächlich Wasser und das gewünschte Diamin enthält, kann diese in die Destillation zurückgeführt werden.

Ein weiterer Gegenstand der vorliegenden Erfindung ist eine Vorrichtung zur Herstellung von Diaminen enthaltend
(a) einen ersten Reaktor zur Addition eines Monoamins an ein Alkenylnitril, der zumindest mit
   i) einem oder mehreren getrennten oder gemeinsamen Einlässen für das Monoamin, das Alkenylnitril und Wasser;
   ii) mindestens einem ersten Auslass, der geeignet ist, Dampf, der im Wesentlichen aus Wasser und dem Monoamin besteht, aus dem ersten Reaktor abzuführen; und
   iii) mindestens einem zweiten Auslass, der geeignet ist, das gebildete Aminoalkylnitril im Sumpf abzuführen;
   versehen ist;
(b) einen zweiten Reaktor zur batchweisen Hydrierung des Aminoalkylnitrils, der zumindest mit
   i) mindestens einem ersten Einlass, der mit dem zweiten Auslass des ersten Reaktors verbunden ist;
   ii) einem oder mehreren weiteren getrennten oder gemeinsamen Einlässen für Wasserstoff, Katalysator, Base, Wasser sowie einem Inertgas (vorzugsweise Stickstoff); sowie
   iii) einem Auslass zur Abführung des Reaktionsproduktes
   versehen ist; und
(c) einer Vorrichtung zur Gewinnung des Diamins aus dem Reaktionsprodukt, die zumindest mit
   i) mindestens einem Einlass, der mit dem Auslass des zweiten Reaktors verbunden ist, sowie
   ii) mindestens einem Auslass, der geeignet ist, das gewonnene Diamin abzuführen,
   versehen ist.

Vorzugsweise ist der erste Auslass des ersten Reaktors mit einem Einlass eines Zwischenbehälters verbunden, der wiederum mindestens einen Auslass aufweist, der die Rückführung der abgedampften, gegebenenfalls kondensierten Brüden aus dem ersten Reaktor in diesen ermöglicht.

Weiterhin ist bevorzugt, dass die Verbindung zwischen erstem und zweitem Reaktor einen Sammelbehälter sowie einen ersten Filter enthält. Dieser ist jedoch nicht zwingend erforderlich.

Ebenso ist es bevorzugt, dass der zweite Reaktor mindestens einen weiteren Auslass aufweist, um sedimentierten oder zu sedimentierenden Katalysator abzuführen.

Weiterhin ist es bevorzugt, dass die Verbindung zwischen zweitem Reaktor und der Vorrichtung zur Gewinnung des Diamins einen zweiten Filter aufweist, um zu vermeiden, dass Katalysatormenge in die Vorrichtung zur Gewinnung des Diamins gelangt. Die Rückspülung des Katalysators im zweiten Filter erfolgt vorzugsweise während der Zuführung des Aminoalkylnitrils.

Der zweite Filter kann gleich dem ersten Filter oder beide Filter können ebenso identisch sein. Im Fall, dass nur ein Filter verwendet wird, können hierdurch Kosten vermieden werden.

Die Vorrichtung zur Gewinnung des Diamins kann beispielsweise eine Destillationskolonne oder eine Rektifikationskolonne sein. Bevorzugt ist der Destillationskolonne eine Destillationsblase dahingehend vorgeschaltet, dass zwischen der Verbindung von zweitem Reaktor und der Vorrichtung zur Gewinnung des Diamins eine solche Destillationsblase enthalten ist.

Ein weiterer Gegenstand der vorliegenden Erfindung ist die Verwendung einer Vorrichtung gemäß der vorliegenden Erfindung zur Herstellung eines Diamins durch Umsetzung eines Alkenylnitrils mit einem Monoamin und anschließende Hydrierung, wobei vorzugsweise ein erfindungsgemäßes Verfahren, wie dies oben ausgeführt wurde, eingesetzt wird.

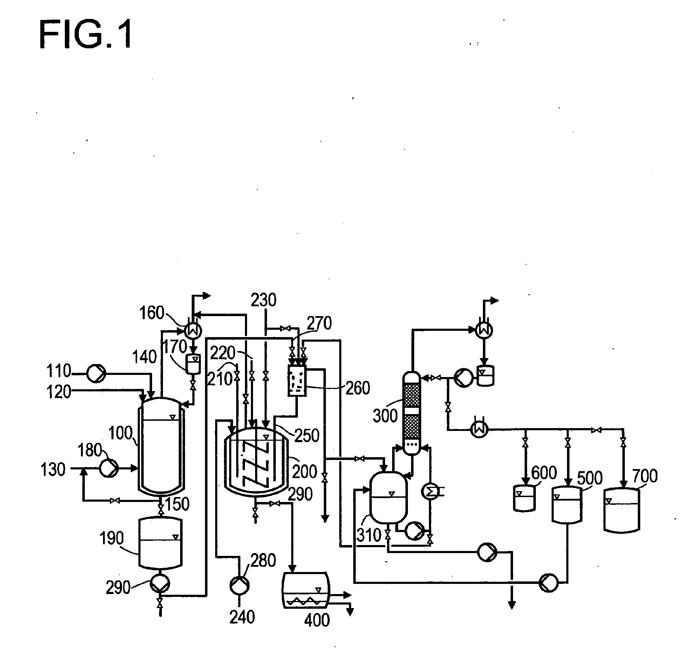

Die nachfolgenden Beispiele sowie Figur 1 erläutern die Erfindung näher, ohne diese einzuschränken. Hierbei zeigt Figur 1 eine erfindungsgemäße Vorrichtung. Die Beispiele beziehen sich auf die Synthese von DMAPA ausgehend von DMA sowie ACN.

### Beispiele

### 1. DMAPN-Synthese

### 1.1 Prozedur im Normalbetrieb

Die Synthese läuft diskontinuierlich in zwei parallel betriebenen Blasensäulen 100 von je 9,1 m³ mit Rückflusskühler 160 (Wt-Fläche 75 m²) bei 5-1 bar unter Siedekühlung und Sekundär-Kühlwasser-Kühlung über Doppelmantel bei <100°C. Kühlmedium in Kühler 160 ist Sole (T_{Ein} -5°C, T_{Aus} 0°C), das Sekundär-Kühlwasser hat eine Eintrittstemperatur von 34°C und eine Austrittstemperatur von 43°C. Es werden insgesamt DMA (2914 kg) über Einlass 110 und Wasser (124 kg) über Einlass 120, (davon 2727 kg frisches DMA und 107 kg Frischwasser, Rest aus Zwischenbehälter 170), vorgelegt und über 2,5 h ACN (3108 kg) über eine Düse von Einlass 130 zugepumpt. An der Eintrittstelle führt die Reaktionswärme zur Verdampfung des flüssigen DMA, was zur Durchmischung des Reaktionsgemisches in der Blasensäule beiträgt. Verdampftes DMA wird über Auslass 140 im Rückflusskühler kondensiert. Über eine temperaturgesteuerte Druckregelung wird im Verlauf der Reaktion der Druck von 5 auf 1 bar abgesenkt, so dass bei konstanter Temperatur bzw. unterhalb maximal 100°C DMA verdampft werden kann. Nach Eindüsen der ACN-Gesamtmenge wird die Zulaufpumpe 180 auf Kreislauf umgestellt, um den Umsatz zu vervollständigen. Nach 1 h Nachreaktion wird der Reaktorinhalt über den Doppelmantel mit 4 bar Dampf (151°C) aufgeheizt, wobei überwiegend DMA und zum Teil Wasser und DMAPN (insgesamt 308 kg, davon 204 kg DMA, 16 kg Wasser und 88 kg DMAPN) verdampft, kondensiert, und im Behälter 170 bis zur Verwendung im nächsten Batch zwischengespeichert werden. Wenn die Sumpftemperatur auf 130°C ansteigt, ist der Abdampfvorgang beendet. Die Reaktorfüllung (5942 kg), die noch ca. 1,1% DMA und 1,8% Wasser enthält, wird abgekühlt und in den Lagertank 190 über Auslass 150 abgelassen.

### 2. DMAPA Synthese

### 2.1 Allgemeines

Die Hydrierung wird in zwei parallelen Doppelmantelreaktoren 200 mit einem Volumen von je 32 m³, ausgestattet mit einem dreistufigen Schrägblattrührer mit einer Motorenleistung von je 120 kW durchgeführt. Kühlmedium ist Sekundär-Kühlwasser. Die Batchzeit beträgt 16 h und ein Ansatz beinhaltet 15820 kg Roh-DMAPN aus der Anlagerung (97,1 % Gehalt).

### 2.2 Anfahren des Hydrierreaktors mit frischer Katalysatorfüllung

Vor der ersten Hydrierung oder nach einer Totalentleerung des Kessels werden über die Pumpe 280 und Einlass 240 Katalysator Raney^{™}-Ni (500 kg als 50%ige Suspension in Wasser) und wässrige KOH (60 kg als 25%ige Lösung), sowie über Pumpe 290 rohes oder reines DMAPA (4 m³) im Reaktor vorgelegt und angemaischt. Der Reaktor wird 2 x mit Stickstoff über Einlass 220 auf 10 bar aufgepresst und dann auf 1 bar entspannt. Das weitere Vorgehen ist unter 2.3 beschrieben.

### 2.3 Hydrierung

Vor dem Beginn der Hydrierung steht der Reaktor immer unter Stickstoff (s. 2.2 bzw. unten) Zum Austausch von Stickstoff gegen Wasserstoff über Einlass 210 wird der Reaktor unter Rühren 2 x mit Wasserstoff über Einlass 210 auf 10 bar aufgepresst und jedes Mal wieder auf 1 bar entspannt. Nach dem Gaswechsel von Stickstoff auf Wasserstoff wird ein Wasserstoffdruck von 10 bar eingestellt. Unter beständigem Rühren wird die Katalysatormaische nun auf 90°C aufgeheizt (Doppelmantel, 1 bar Dampf). Bei Erreichen dieser Temperatur wird auf 30 bar Wasserstoff aufgepresst und mit der Zudosierung von Roh-DMAPN aus dem Behälter 190 mittels Pumpe 290 über den Filter 260 und Einlass 250 begonnen. Dabei wird von Heizen auf Kühlen umgestellt. Das rohe DMAPN (15820 kg) wird über 9 h zum Katalysator zugefahren, Wasserstoff wird druckgeregelt nachgepresst. Nach 9 h wird noch 1 h nachhydriert. Wenn die Wasserstoffaufnahme beendet ist, wird auf Normaldruck entspannt (Fackel) und die Rührung ausgeschaltet, so dass sich der Katalysator absetzt. Anschließend wird der Reaktorinhalt über eine Steigleitung durch den Sintermetallfilter 260 bei 90°C mittels Stickstoff in die Destillationsblase 310 gedrückt. Der Reaktorinhalt enthält 95,2% DMAPA. Es wird ein Füllvolumen von ca. 5 m³ belassen, in dem sich der Hauptteil des Katalysators befindet.

Abfiltriertes Raney-Nickel in Reaktor 200 wird bei der Eduktdosierung des nächsten Ansatzes in den Hydrierreaktor zurückgespült. Vor jedem Batch wird KOH (6 kg als 25%ige Lösung in Wasser) zugegeben, und vor jedem 10. Batch werden zusätzlich Raney^{™}-Ni (50 kg als 50%ige Suspension in Wasser) und KOH (12 kg als 25%ige wässrige Lösung) zugefahren. Dadurch wird nach 160 Batches eine Gesamtmenge von etwa 650 kg Katalysator erreicht, was einer Anfangskonzentration von ca. 14% entspricht.

Der Filter 260 wird beim Zudosieren des rohen DMAPNs automatisch zurückgespült, kann bei Bedarf jedoch auch aus Blase 310 mit rohem DMAPA oder mit Wasser von der Rohrbrücke gespült werden.

### 3. Katalysatorwechsel

Nach 160 Batches (alle vier Monate) wird der Katalysator totalentleert und der Hydrierreaktor wieder neu angefahren. Dazu wird der Kesselinhalt wie üblich über die Steigleitung entleert, dann mit 4 m³ Wasser wieder aufgefüllt, gerührt, der Katalysator absitzen gelassen, und die überstehende DMAPA / Wasser-Waschlösung über den Filter 260 herausgefahren. Dieser Schritt wird dreimal wiederholt. Anschließend wird noch zweimal nachgewaschen, jedoch die Waschlösung über den Filter ins Abwasser gedrückt. Nach insgesamt 5 Waschschritten wird der Katalysator über das Bodenventil 290 in den Absetzbehälter 400 abgelassen, wo er sedimentiert und mittels Schnecke in Fässer (Recycling der Anlieferungsfässer) abgegeben wird. Der Kessel wird noch mit 5 m³ Wasser nachgespült.

Die wasserhaltige Waschlösung wird aufdestilliert. Der durch das Wasser mengenmäßig stark zunehmende Zwischenlauf, der normalerweise in den Behälter 500 zwischengespeichert wird, wird nicht zurückgeführt, sondern verbrannt oder ins Abwasser gegeben.

### 4. Destillation

Die Destillation ist eine Batch-Destillation in der Kolonne 300 bei Normaldruck und einer Sumpftemperatur von 90 bis maximal 150°C. Die Batchzeit beträgt 16 h, ein Batch umfasst 16500 kg Roh-DMAPA. Zuerst wird Abgas abgezogen, (200 kg, davon 16 kg NH₃, 160 kg DMA und 24 kg Wasser), dann werden Leichtsieder (550 kg, vor allem DMA und Wasser) (in Behälter 600), ein wasserhaltiger Zwischenlauf, der in die Destillation zurückgeht (in Behälter 500), und der Reinlauf mit 99,5% DMAPA (15000 kg) (in Behälter 700), abdestilliert und in den entsprechenden Behältern 500, 600 und 700 gelagert. Im Sumpf (650 kg) verbleiben 365 kg DMAPA, 267 kg Bis-DMAPA und 18 kg DMAPN, die verbrannt werden.

## Patentansprüche

1. Verfahren zur großtechnischen Herstellung eines Diamins ausgehend von einem entsprechenden Alkenylnitril, das mindestens eine C-C-Doppelbindung enthält, die Schritte enthaltend
(a) Umsetzung des Alkenylnitrils in einem ersten Reaktor mit einem entsprechenden Monoamin unter exothermer Addition des Monoamins an die mindestens eine Doppelbindung zu einem Aminoalkylnitril, wobei das Monoamin sowie Wasser vorgelegt werden und das Alkenylnitril hinzugefahren wird;
(b) Abdampfen nicht umgesetzten Alkenylnitrils und Monoamins zur Anreicherung des Aminoalkylnitril-Produktes im Sumpf des ersten Reaktors;
(c) Überführung des Aminoalkylnitril-Sumpfproduktes aus Schritt (b) in einen zweiten Reaktor;
(d) Batchweise katalytische Hydrierung des in Schritt (c) überführten Aminoalkylnitrils zum Diamin in dem zweiten Reaktor, wobei für jeden Batch ein für die Hydrierung von Nitrilen zu Aminen geeigneter Katalysator sowie Wasser, das gewünschte Diamin und eine Base vorgelegt, der zweite Reaktor mit Wasserstoff beaufschlagt und das in Schritt (c) überführte Aminoalkylnitril zudosiert wird; und
(e) Gewinnung des Diamins und gegebenenfalls Wiederholung der Schritte (a) bis (e).

2. Verfahren nach Anspruch 1, **dadurch gekennzeichnet, dass** das Alkenylnitril Acrylsäurenitril (ACN) ist.

3. Verfahren nach Anspruch 1 oder 2, **dadurch gekennzeichnet, dass** das Monoamin Dimethylamin (DMA) ist.

4. Verfahren nach einem der Ansprüche 1 bis 3, **dadurch gekennzeichnet, dass** das Diamin 3-Dimethylaminopropylamin (DMAPA) ist.

5. Verfahren nach einem der Ansprüche 1 bis 4, **dadurch gekennzeichnet, dass** in Schritt (a) während der gesamten Umsetzung im Reaktionsgemisch das Monoamin in einem stöchiometrischen Überschuss zum Alkenylnitril von mindestens 1 mol-% vorliegt.

6. Verfahren nach einem der Ansprüche 1 bis 5, **dadurch gekennzeichnet, dass** der erste Reaktor eine Blasensäule darstellt.

7. Verfahren nach einem der Ansprüche 1 bis 6, **dadurch gekennzeichnet, dass** die Umsetzung in Schritt (a) unter Siedehitze unter Rückführung des Amins erfolgt.

8. Verfahren nach einem der Ansprüche 1 bis 7, **dadurch gekennzeichnet, dass** die Sumpftemperatur in Schritt (a) unter 130°C gehalten wird.

9. Verfahren nach Anspruch 8, **dadurch gekennzeichnet, dass** die Sumpftemperatur durch temperaturgesteuerte Druckabsenkung im ersten Reaktor konstant gehalten wird.

10. Verfahren nach einem der Ansprüche 1 bis 9, **dadurch gekennzeichnet, dass** die in Schritt (b) erhaltenen Brüden zumindest teilweise in einer nachfolgenden Umsetzung wieder für einen Schritt (a) vorgelegt werden.

11. Verfahren nach einem der Ansprüche 1 bis 10, **dadurch gekennzeichnet, dass** bei der Überführung des Aminoalkylnitrils in Schritt (c) außer einem Filtrationsschritt keine weiteren Reinigungsschritte erfolgen.

12. Verfahren nach einem der Ansprüche 1 bis 11, **dadurch gekennzeichnet, dass** das in Schritt (c) überführte Aminoalkylnitril jeweils höchstens 5 Gew.-% an Monoamin und Wasser enthält.

13. Verfahren nach einem der Ansprüche 1 bis 12, **dadurch gekennzeichnet, dass** in Schritt (d) der Katalysator im Reaktionsgemisch zu mindestens 1 Gew.-% bezogen auf das insgesamt zugegebene Aminoalkylnitril vorhanden ist.

14. Verfahren nach einem der Ansprüche 1 bis 13, **dadurch gekennzeichnet, dass** der Katalysator Nickel enthält.

15. Verfahren nach einem der Ansprüche 1 bis 14, **dadurch gekennzeichnet, dass** in Schritt (d) für einen oder mehrere nachfolgende Chargen (batches) Katalysatormenge zur im zweiten Reaktor befindlichen Katalysatormenge zugegeben wird.

16. Verfahren nach einem der Ansprüche 1 bis 15, **dadurch gekennzeichnet, dass** die Schritte (a) bis (e) mindestens 50-mal wiederholt werden, bevor der Katalysator ausgetauscht wird.

17. Verfahren nach einem der Ansprüche 1 bis 16, **dadurch gekennzeichnet, dass** die Base Kaliumhydroxid, Natriumhydroxid oder eine Mischung davon ist.

18. Verfahren nach einem der Ansprüche 1 bis 17, **dadurch gekennzeichnet, dass** die Gewinnung in Schritt (e) destillativ erfolgt.

19. Verfahren nach Anspruch 18, **dadurch gekennzeichnet, dass** vor der Destillation das Reaktionsprodukt filtriert und in eine Destillationskolonne überführt wird.

20. Verfahren nach Anspruch 19, **dadurch gekennzeichnet, dass** vor der Filtration der Hauptteil des Katalysators sedimentativ abgetrennt wird.

21. Verfahren nach einem der Ansprüche 18 bis 20, **dadurch gekennzeichnet, dass** eine Vorlauffraktion hauptsächlich bestehend aus Wasser und Monoamin in den ersten Reaktor zurückgeführt werden.

22. Verfahren nach einem der Ansprüche 18 bis 21, **dadurch gekennzeichnet, dass** eine Vorlauffraktion, hauptsächlich bestehend aus Wasser und dem gewünschten Diamin, in die Destillation zurückgeführt wird.

23. Vorrichtung zur Herstellung von Diaminen enthaltend
(a) einen ersten Reaktor zur Addition eines Monoamins an ein Alkenylnitril, der zumindest mit
i) einem oder mehreren getrennten oder gemeinsamen Einlässen für das Monoamin, das Alkenylnitril und Wasser;
ii) mindestens einem ersten Auslass, der geeignet ist, Dampf, der im Wesentlichen aus Wasser und dem Monoamin besteht, aus dem ersten Reaktor abzuführen; und
iii) mindestens einem zweiten Auslass, der geeignet ist, das gebildete Aminoalkylnitril im Sumpf abzuführen;
versehen ist;
(b) einen zweiten Reaktor zur batchweisen Hydrierung des Aminoalkylnitrils, der zumindest mit
i) mindestens einem ersten Einlass, der mit dem zweiten Auslass des ersten Reaktors verbunden ist;
ii) einem oder mehreren weiteren getrennten oder gemeinsamen Einlässen für Wasserstoff, Katalysator, Base, Wasser sowie einem Inertgas; sowie
iii) einem Auslass zur Abführung des Reaktionsproduktes
versehen ist; und
(c) einer Vorrichtung zur Gewinnung des Diamins aus dem Reaktionsprodukt, die zumindest mit
i) mindestens einem Einlass, der mit dem Auslass des zweiten Reaktors verbunden ist, sowie
ii) mindestens einem Auslass, der geeignet ist, das gewonnene Diamin abzuführen,
versehen ist.

24. Vorrichtung nach Anspruch 23, **dadurch gekennzeichnet, dass** der erste Auslass des ersten Reaktors mit einem Einlass eines Zwischenbehälter verbunden ist, der wiederum mindestens einen Auslass aufweist, der die Rückführung der abgedampften gegebenenfalls kondensierten Brüden aus dem ersten Reaktor in diesen ermöglicht.

25. Vorrichtung nach Anspruch 23 oder 24, **dadurch gekennzeichnet, dass** die Verbindung zwischen erstem und zweitem Reaktor einen Sammelbehälter sowie einen ersten Filter enthält.

26. Vorrichtung nach einem der Ansprüche 23 bis 25, **dadurch gekennzeichnet, dass** der zweite Reaktor mindestens einen weiteren Auslass aufweist, über den sedimentierter Katalysator abgeführt werden kann.

27. Vorrichtung nach einem der Ansprüche 23 bis 26, **dadurch gekennzeichnet, dass** die Verbindung zwischen zweitem Reaktor und Vorrichtung zur Gewinnung des Diamins einen zweiten Filter aufweist.

28. Vorrichtung nach Anspruch 25 und 27, **dadurch gekennzeichnet, dass** erster und zweiter Filter identisch sind.

29. Vorrichtung nach einem der Ansprüche 23 bis 28, **dadurch gekennzeichnet, dass** die Vorrichtung zur Gewinnung des Diamins eine Destillationskolonne ist.

30. Vorrichtung nach einem der Ansprüche 23 bis 29, **dadurch gekennzeichnet, dass** der Destillationskolonne eine Destillationsblase vorgeschaltet ist.

31. Verwendung einer Vorrichtung nach einem der Ansprüche 23 bis 30 zur Herstellung eines Diamins durch Umsetzung eines. Alkenylnitrils mit einem Monoamin und anschließender Hydrierung, insbesondere nach einem Verfahren gemäß einem der Ansprüche 1 bis 22.

## Claims

1. A process for the industrial preparation of a diamine starting from a corresponding alkenyl nitrile comprising at least one C-C double bond, which comprises the steps
(a) reaction of the alkenyl nitrile with a corresponding monoamine in a first reactor so that the monoamine adds exothermically onto the at least one double bond to form an aminoalkyl nitrile, with the monoamine and water being charged initially and the alkenyl nitrile being fed in;
(b) evaporation of unreacted alkenyl nitrile and monoamine to increase the concentration of the aminoalkyl nitrile product in the bottoms of the first reactor;
(c) transfer of the aminoalkyl nitrile bottom product from step (b) to a second reactor;
(d) batchwise catalytic hydrogenation of the aminoalkyl nitrile transferred in step (c) to the diamine in the second reactor, with each batch being obtained by initially charging a catalyst suitable for the hydrogenation of nitriles to amines and also water, the desired diamine and a base, introducing hydrogen into the second reactor and feeding in the aminoalkyl nitrile transferred in step (c); and
(e) isolation of the diamine and, optionally, repetition of the steps (a) to (e).

2. The process according to claim 1, wherein the alkenyl nitrile is acrylonitrile (ACN).

3. The process according to claim 1 or 2, wherein the monoamine is dimethylamine (DMA).

4. The process according to any of claims 1 to 3, wherein the diamine is 3-dimethylaminopropylamine (DMAPA).

5. The process according to any of claims 1 to 4, wherein the monoamine is present in a stoichiometric excess relative to the alkenyl nitrile of at least 1 mol% in the reaction mixture during the entire reaction in step (a).

6. The process according to any of claims 1 to 5, wherein the first reactor is a bubble column.

7. The process according to any of claims 1 to 6, wherein the reaction in step (a) is carried out at the boiling point with recirculation of the amine.

8. The process according to any of claims 1 to 7, wherein the temperature of the bottoms in step (a) is kept below 130°C.

9. The process according to claim 8, wherein the temperature of the bottoms is kept constant in the first reactor by means of a temperature-controlled pressure reduction.

10. The process according to any of claims 1 to 9, wherein the vapor obtained in step (b) is at least partly reused for the initial charge for step (a) in a subsequent reaction.

11. The process according to any of claims 1 to 10, wherein no further purification steps apart from a filtration step are carried out during the transfer of the aminoalkyl nitrile into step (c).

12. The process according to any of claims 1 to 11, wherein the aminoalkyl nitrile transferred in step (c) comprises not more than 5% by weight of monoamine and not more than 5% by weight of water.

13. The process according to any of claims 1 to 12, wherein the catalyst is present in the reaction mixture in step (d) in an amount of at least 1% by weight based on the total aminoalkyl nitrile added.

14. The process according to any of claims 1 to 13, wherein the catalyst comprises nickel.

15. The process according to any of claims 1 to 14, wherein, in step (d), an amount of catalyst sufficient for one or more of the subsequent batches is added to the amount of catalyst present in the second reactor.

16. The process according to any of claims 1 to 15, wherein the steps (a) to (e) are repeated at least 50 times before the catalyst is replaced.

17. The process according to any of claims 1 to 16, wherein the base is potassium hydroxide, sodium hydroxide or a mixture thereof.

18. The process according to any of claims 1 to 17, wherein the isolation in step (e) is carried out by distillation.

19. The process according to claim 18, wherein the reaction product is filtered and transferred to a distillation column prior to the distillation.

20. The process according to claim 19, wherein the major part of the catalyst is separated off by sedimentation prior to the filtration.

21. The process according to any of claims 18 to 20, wherein a first fraction consisting mainly of water and monoamine is recirculated to the first reactor.

22. The process according to any of claims 18 to 21, wherein a first fraction consisting mainly of water and the desired diamine is recirculated to the distillation.

23. An apparatus for preparing diamines, which comprises
(a) a first reactor for addition of a monoamine onto an alkenyl nitrile, which is provided with at least
i) one or more separate or joined inlets for the monoamine, the alkenyl nitrile and water;
ii) at least one first outlet which is suitable for discharging vapor which consists essentially of water and the monoamine from the first reactor; and
iii) at least one second outlet which is suitable for discharging the aminoalkyl nitrile formed in the bottoms;
(b) a second reactor for the batchwise hydrogenation of the aminoalkyl nitrile, which is provided with at least
i) at least one first inlet which is connected to the second outlet of the first reactor;
ii) one or more further separate or joined inlets for hydrogen, catalyst, base, water and an inert gas; and
iii) an outlet for discharge of the reaction product; and
(c) an apparatus for isolating the diamine from the reaction product, which is provided with at least
i) at least one inlet which is connected to the outlet of the second reactor, and
ii) at least one outlet which is suitable for discharging the diamine isolated.

24. The apparatus according to claim 23, wherein the first outlet of the first reactor is connected to an inlet of an intermediate container which in turn has at least one outlet which makes it possible for the evaporated, optionally condensed vapor from the first reactor to be recirculated to this.

25. The apparatus according to claim 23 or 24, wherein the connection between the first and second reactors comprises a collection vessel and a first filter.

26. The apparatus according to any of claims 23 to 25, wherein the second reactor has at least one further outlet via which the sedimented catalyst can be discharged.

27. The apparatus according to any of claims 23 to 26, wherein the connection between the second reactor and the apparatus for isolating the diamine has a second filter.

28. The apparatus according to either of claims 25 and 27, wherein the first and second filters are identical.

29. The apparatus according to any of claims 23 to 28, wherein the apparatus for isolating the diamine is a distillation column.

30. The apparatus according to any of claims 23 to 29, wherein the distillation column is preceded by a distillation pot.

31. The use of an apparatus according to any of claims 23 to 30, for preparing a diamine by reaction of an alkenyl nitrile with a monoamine and subsequent hydrogenation, in particular by a process according to any of claims 1 to 22.

## Revendications

1. Procédé pour la production industrielle d'une diamine à partir d'un alcénylnitrile correspondant, qui comporte au moins une double liaison C-C, les étapes comprenant
(a) mise en réaction de l'alcénylnitrile dans un premier réacteur avec une monoamine correspondante, avec fixation par addition exothermique de la monoamine sur ladite au moins une double liaison, conduisant à un aminoalkylnitrile, en disposant au préalable la monoamine ainsi que l'eau et en y envoyant l'alcénylnitrile ;
(b) évaporation de l'alcénylnitrile et de la monoamine n'ayant pas réagi, pour la concentration du produit aminoalkylnitrile dans la zone inférieure du premier réacteur ;
(c) transfert du produit aminoalkylnitrile résiduel de l'étape (b) dans un deuxième réacteur ;
(d) hydrogénation catalytique en mode discontinu de l'aminoalkylnitrile transféré dans l'étape (c), conduisant à la diamine, dans le deuxième réacteur, en disposant au préalable pour chaque charge un catalyseur approprié à l'hydrogénation de nitriles en amines ainsi que de l'eau, la diamine recherchée et une base, le deuxième réacteur étant alimenté en hydrogène et l'aminoalkylnitrile transféré dans l'étape (c) étant ajouté par addition dosée ; et
(e) récupération de la diamine et éventuellement répétition de étapes (a) à (e).

2. Procédé selon la revendication 1, **caractérisé en ce que** l'alcénylnitrile est l'acrylonitrile (ACN).

3. Procédé selon la revendication 1 ou 2, **caractérisé en ce que** la monoamine est la diméthylamine (DMA).

4. Procédé selon l'une quelconque des revendications 1 à 3, **caractérisé en ce que** la diamine est la 3-diméthylaminopropylamine (DMAPA).

5. Procédé selon l'une quelconque des revendications 1 à 4, **caractérisé en ce que** dans l'étape (a) la monoamine est présente pendant toute la réaction dans le mélange réactionnel en un excès stoechiométrique d'au moins 1 % en moles par rapport à l'alcénylnitrile.

6. Procédé selon l'une quelconque des revendications 1 à 5, **caractérisé en ce que** le premier réacteur est une colonne à barbotage.

7. Procédé selon l'une quelconque des revendications 1 à 6, **caractérisé en ce que** la réaction dans l'étape (a) s'effectue à la température d'ébullition avec recirculation de l'amine.

8. Procédé selon l'une quelconque des revendications 1 à 7, **caractérisé en ce que** dans l'étape (a) on maintient la température dans la zone inférieure au-dessous de 130 °C.

9. Procédé selon la revendication 8, **caractérisé en ce que** dans le premier réacteur on maintient constante la température dans la zone inférieure par abaissement de la pression commandé par la température.

10. Procédé selon l'une quelconque des revendications 1 à 9, **caractérisé en ce que** dans une réaction subséquente on dispose au préalable à nouveau pour une étape (a) au moins en partie les vapeurs obtenues dans l'étape (b).

11. Procédé selon l'une quelconque des revendications 1 à 10, **caractérisé en ce qu'**hormis une étape de filtration aucune autre étape de purification n'est effectuée lors du transfert de l'aminoalkyinitrile dans l'étape (c).

12. Procédé selon l'une quelconque des revendications 1 à 11, **caractérisé en ce que** l'aminoalkylnitrile transféré dans l'étape (c) contient au maximum 5 % en poids, respectivement, de monoamine et d'eau.

13. Procédé selon l'une quelconque des revendications 1 à 12, **caractérisé en ce que** dans l'étape (d) le catalyseur est présent dans le mélange réactionnel à raison d'au moins 1 % en poids, par rapport à l'aminoalkylnitrile ajouté au total.

14. Procédé selon l'une quelconque des revendications 1 à 13, **caractérisé en ce que** le catalyseur contient du nickel.

15. Procédé selon l'une quelconque des revendications 1 à 14, **caractérisé en ce que** dans l'étape (d) on ajoute une quantité de catalyseur pour une ou plusieurs charges (batches) subséquentes à la quantité de catalyseur se trouvant dans le deuxième réacteur.

16. Procédé selon l'une quelconque des revendications 1 à 15, **caractérisé en ce qu'**avant de changer le catalyseur on répète au moins 50 fois les étapes (a) à (e).

17. Procédé selon l'une quelconque des revendications 1 à 16, **caractérisé en ce que** la base est l'hydroxyde de potassium, l'hydroxyde de sodium ou un mélange de ceux-ci.

18. Procédé selon l'une quelconque des revendications 1 à 17, **caractérisé en ce que** la récupération dans l'étape (e) s'effectue par distillation.

19. Procédé selon la revendication 18, **caractérisé en ce qu'**avant la distillation on filtre le produit de réaction et on le transfère dans une colonne de distillation.

20. Procédé selon la revendication 19, **caractérisé en ce qu'**avant la filtration on sépare la majeure partie du catalyseur par sédimentation.

21. Procédé selon l'une quelconque des revendications 18 à 20, **caractérisé en ce qu'**on recycle dans le premier réacteur une fraction de tête principalement constituée d'eau et de monoamine.

22. Procédé selon l'une quelconque des revendications 18 à 21, **caractérisé en ce qu'**on recycle dans la distillation une fraction de tête principalement constituée d'eau et de la diamine recherchée.

23. Dispositif destiné à la production de diamines, comportant
(a) un premier réacteur destiné à la fixation par addition d'une monoamine sur un alcénylnitrile, qui est au moins muni
i) d'une ou plusieurs entrées séparées ou communes pour la monoamine, l'alcénylnitrile et l'eau ;
ii) d'au moins une première sortie qui est appropriée à évacuer du premier réacteur la vapeur qui est essentiellement constituée d'eau et de la monoamine ;
iii) d'au moins une deuxième sortie qui est appropriée à évacuer dans la zone inférieure l'aminoalkylnitrile formé ;
(b) un deuxième réacteur destiné à l'hydrogénation en mode discontinu de l'aminoalkylnitrile, qui est au moins muni
i) d'au moins une première entrée qui est raccordée à la deuxième sortie du premier réacteur ;
ii) d'une ou plusieurs entrée séparées ou communes pour hydrogène, catalyseur, base, eau ainsi qu'un gaz inerte : ainsi que
iii) d'une sortie pour l'évacuation du produit de réaction ; et
(c) un dispositif destiné à la récupération de la diamine à partir du produit de réaction, qui est au moins muni
i) d'au moins une entrée qui est raccordée à la sortie du deuxième réacteur, ainsi que
ii) d'au moins une sortie qui est appropriée à évacuer la diamine recueillie.

24. Dispositif selon la revendication 23, **caractérisé en ce que** la première sortie du premier réacteur est raccordée à une entrée d'un récipient intermédiaire qui à son tour comporte au moins une sortie qui permet le renvoi des vapeurs produites, éventuellement condensées, du premier réacteur dans ce dernier.

25. Dispositif selon la revendication 23 ou 24, **caractérisé en ce que** le raccordement entre le premier réacteur et le deuxième comporte un récipient collecteur ainsi qu'un premier filtre.

26. Dispositif selon l'une quelconque des revendications 23 à 25, **caractérisé en ce que** le deuxième réacteur comporte au moins une autre sortie, par laquelle le catalyseur sédimenté peut être évacué.

27. Dispositif selon l'une quelconque des revendications 23 à 26, **caractérisé en ce que** le raccordement entre deuxième réacteur et dispositif destiné à la récupération de la diamine comporte un deuxième filtre.

28. Dispositif selon les revendications 25 et 27, **caractérisé en ce que** les premier et deuxième filtres sont identiques.

29. Dispositif selon l'une quelconque des revendications 23 à 28, **caractérisé en ce que** le dispositif destiné à la récupération de la diamine est une colonne de distillation.

30. Dispositif selon l'une quelconque des revendications 23 à 29, **caractérisé en ce qu'**un vase de distillation est placé en amont de la colonne de distillation.

31. Utilisation d'un dispositif selon l'une quelconque des revendications 23 à 30, pour la production d'une diamine par mise en réaction d'un alcénylnitrile avec une monoamine et hydrogénation subséquente, en particulier conformément à un procédé selon l'une quelconque des revendications 1 à 22.
